# EUROPEAN PATENT APPLICATION

(11) **EP 4 755 300 A1**
(43) Date of publication of application: **10.06.2026**
(21) Application number: 25747667.1
(22) Date of filing: 21.01.2025
(51) Int. Cl.: A61B 5/145

(54) **SENSOR APPLICATION DEVICE**

(30) Priority: 02.02.2024 CN 202410151897
(71) Applicant: SHENZHEN SISENSING CO., LTD., Shenzhen Guangdong 518110 (CN)
(72) Inventor: LI, Yunfeng, Shenzhen, Guangdong 518110 (CN)
(74) Representative: Isern Patentes y Marcas S.L.
(86) International application number: PCT/CN2025/073763
(87) International publication number: WO 2025/162095

(57) **Abstract**

A sensor applicator is provided in the present disclosure, the sensor applicator is configured to apply a sensor control device to a host, the sensor applicator having a proximal end and a distal end and comprising a housing, a sensor control device carrier, a sharp module, and a trigger member; wherein the sensor control device carrier is configured to releasably receive the sensor control device; the sharp module is configured to releasably apply the sensor control device to the host; the trigger member is configured to, when triggered by the housing, cause the sensor control device carrier to drive the sharp module and the sensor control device to move from the distal end to the proximal end; and the sharp module is further configured to, when moving a predetermined distance relative to the trigger member under the drive of the sensor control device carrier, be triggered by the trigger member and move from the proximal end to the distal end. According to the present disclosure, a sensor applicator capable of improving triggering effectiveness and operator experience can be provided.

## Description

### Technical Field

The present disclosure relates to the field of biomedical engineering, and in particular, to a sensor applicator.

### Background of the Invention

For diabetic patients, it is essential to monitor glucose levels in bodily fluids in real-time and continuously around the clock. This enables patients to promptly regulate their glucose concentration through methods such as dietary adjustments or medication, thereby reducing and minimizing the occurrence of complications arising from abnormal glucose levels. Traditional methods for monitoring glucose levels in bodily fluids require patients to perform repeated fingertip blood sampling, which is not only inconvenient but also causes significant discomfort. In contrast, CGM (Continuous Glucose Monitoring) is a device capable of dynamically monitoring human glucose concentrations, thereby eliminating the need for repeated fingertip blood sampling.

Prior to the commencement of normal operation of a CGM, the CGM must first be applied to the skin surface of the human body. In current conventional practice, a dedicated sensor applicator is employed to pick up the CGM. The CGM is then applied to the skin surface by pressing a button on the housing of the sensor applicator, thereby enabling continuous and dynamic monitoring of glucose concentration in human bodily fluids.

However, the button-based approach utilized in the prior art results in an excessively small force application area for the operator, which adversely affects the efficacy of force application. Moreover, reliance on a single button for driving the mechanism may, in more severe instances, lead to difficulties in effectively applying the CGM to the skin surface of the human body.

### Summary

The present disclosure is made in view of the above-described circumstances in the prior art, and aims to provide a sensor applicator capable of improving triggering effectiveness and operator experience.

To this end, the present disclosure provides a sensor applicator configured to apply a sensor control device to a host, the sensor applicator having a proximal end and a distal end and comprising a housing, a sensor control device carrier, a sharp module, and a trigger member; wherein the sensor control device carrier is configured to releasably receive the sensor control device; the sharp module is configured to releasably apply the sensor control device to the host; the trigger member is configured to, when triggered by the housing, cause the sensor control device carrier to drive the sharp module and the sensor control device to move from the distal end to the proximal end; and the sharp module is further configured to, when moving a predetermined distance relative to the trigger member under the drive of the sensor control device carrier, be triggered by the trigger member and move from the proximal end to the distal end.

In the sensor applicator according to the present disclosure, when the trigger member is triggered by the housing, the sensor control device carrier is caused to drive the sharp module and the sensor control device to move from the distal end away from the host to the proximal end close to the host. The operator may indirectly trigger the trigger member by triggering the housing, thereby applying the sensor control device to the host. In this configuration, since the housing provides a larger force application area compared to the trigger button in the prior art, the operator can apply force to the housing more effectively by triggering the housing and the trigger member in a coordinated manner. Thereby, triggering effectiveness and operator experience are improved.

Furthermore, in the sensor applicator according to the present disclosure, optionally, the sensor applicator comprises a first driving member configured to drive the sensor control device carrier to move from the distal end to the proximal end when the trigger member is triggered by the housing. In this configuration, driven by the first driving member, the sensor control device carrier drives the sharp module and the sensor control device to move from the distal end to the proximal end, thereby enabling the application of the sensor control device to the host.

Furthermore, in the sensor applicator according to the present disclosure, optionally, the sensor applicator comprises a second driving member configured to drive the sharp module to move from the proximal end to the distal end when the sharp module has moved the predetermined distance relative to the trigger member and is triggered by the trigger member. In this configuration, after the sensor control device is applied to the host, the sharp module can be retracted to the distal end via the drive of the second driving member.

Furthermore, in the sensor applicator according to the present disclosure, optionally, the sensor control device carrier comprises a first locking mechanism and a first triggered mechanism, wherein the first locking mechanism is configured to be locked with the housing in an initial state, and the first triggered mechanism is configured to, when triggered by the trigger member, cause the sensor control device carrier to move from the distal end to the proximal end. In this configuration, via the first locking mechanism, the sensor control device carrier can be locked with the housing in the initial state, reducing the possibility of accidental triggering of the sensor control device carrier. Via the first triggered mechanism, when the sensor control device carrier is triggered by the trigger member, the locked state between the first locking mechanism and the housing is released, thereby allowing the sensor control device carrier to move from the distal end to the proximal end.

Furthermore, in the sensor applicator according to the present disclosure, optionally, the sensor control device carrier comprises a first limiting mechanism, and the sharp module comprises a first retaining mechanism, wherein the first limiting mechanism is configured to restrict movement of the sharp module relative to the sensor control device carrier by engaging with the first retaining mechanism. In this configuration, the first retaining mechanism of the sharp module can be engaged with the first limiting mechanism of the sensor control device carrier, maintaining the sharp module and the sensor control device carrier in a relatively stationary state. Consequently, the sensor control device carrier can drive the sharp module to move together.

Furthermore, in the sensor applicator according to the present disclosure, optionally, the trigger member comprises a first triggering mechanism configured to trigger the first triggered mechanism when the trigger member is triggered by the housing. In this configuration, triggering the first triggered mechanism of the sensor control device carrier by the first triggering mechanism of the trigger member, the locking between the sensor control device carrier and the housing can be released, thereby allowing the sensor control device carrier to move from the distal end to the proximal end.

Furthermore, in the sensor applicator according to the present disclosure, optionally, the trigger member comprises a second triggering mechanism configured to trigger the first retaining mechanism when the sharp module has moved the predetermined distance. In this configuration, when the sharp module has moved the predetermined distance relative to the trigger member and contacts the second triggering mechanism of the trigger member, it can be triggered by the second triggering mechanism, thereby disengaging from the restriction of the sensor control device carrier and moving from the proximal end to the distal end. Thus, the sharp module can be retracted to the distal end after applying the sensor control device to the host.

Furthermore, in the sensor applicator according to the present disclosure, optionally, the trigger member comprises a second triggered mechanism configured to be locked with the housing in the initial state and, when triggered by the housing, cause the first triggering mechanism to trigger the first triggered mechanism. In this configuration, the trigger member can be kept locked with the housing via the second triggered mechanism in the initial state. When the second triggered mechanism is triggered by the housing, the first triggering mechanism of the trigger member is caused to trigger the first triggered mechanism of the sensor control device carrier, thereby releasing the locked state between the first locking mechanism of the sensor control device carrier and the housing, and thus allowing the sensor control device carrier to move from the distal end to the proximal end.

Furthermore, in the sensor applicator according to the present disclosure, optionally, the trigger member extends beyond the housing and is in a first position in the initial state, the housing is in a second position in the initial state, and the housing triggers the trigger member by moving from the second position to the first position. **In** this configuration, moving the housing relative to the trigger member triggers the trigger member, thereby causing the sensor control device carrier to drive the sharp module and the sensor control device to move from the distal end to the proximal end. This facilitates the operator's application of force to the housing to apply the sensor control device to the host, improving triggering effectiveness and operator experience.

Furthermore, in the sensor applicator according to the present disclosure, optionally, the housing comprises a second locking mechanism and a third triggering mechanism, wherein the second locking mechanism is configured to be locked with the first locking mechanism in the initial state, and the third triggering mechanism is configured to be locked with the second triggered mechanism in the initial state and trigger the second triggered mechanism when the housing moves relative to the trigger member. In this configuration, via the second locking mechanism, the housing can be locked with the first locking mechanism of the sensor control device carrier in the initial state, reducing the possibility of accidental triggering of the sensor control device carrier in the initial state. Via the third triggering mechanism, the housing can be locked with the second triggered mechanism of the trigger member in the initial state, reducing the possibility of accidental triggering of the trigger member in the initial state. Furthermore, when the housing moves relative to the trigger member (e.g., when the housing is pressed), the third triggering mechanism on the housing triggers the second triggered mechanism on the trigger member, thereby causing the first triggering mechanism of the trigger member to trigger the first triggered mechanism of the sensor control device carrier, which in turn releases the locked state between the first locking mechanism of the sensor control device carrier and the housing, thereby allowing the sensor control device carrier to move from the distal end to the proximal end.

According to the present disclosure, a sensor applicator capable of improving triggering effectiveness and operator experience can be provided.

### Description of the Drawings

The present disclosure will now be explained in further detail with reference to the drawings, by way of example only.
FIG. 1 is a schematic diagram showing an application scenario of the sensor applicator according to an example of the present disclosure.
FIG. 2 is a schematic external view of the instrument kit according to an example of the present disclosure.
FIG. 3A is a schematic external view of the sensor applicator loaded with a sensor control device according to an example of the present disclosure.
FIG. 3B is an exploded schematic view of FIG. 3A.
FIG. 4 is an exploded schematic view of the instrument kit according to an example of the present disclosure.
FIG. 5A is a schematic perspective view of the sensor control device carrier 13 according to an example of the present disclosure from one viewing angle.
FIG. 5B is a schematic bottom view of FIG. 5A.
FIG. 5C is a schematic top view of FIG. 5A.
FIG. 6 is a schematic perspective view of the sensor control device carrier and the sharp module according to an example of the present disclosure.
FIG. 7 is an exploded schematic view of the sharp module 14 according to an example of the present disclosure.
FIG. 8A is a schematic perspective view of the trigger member according to an example of the present disclosure from one viewing angle.
FIG. 8B is a schematic perspective view of the trigger member according to an example of the present disclosure from another viewing angle.
FIG. 9 is a schematic structural view showing the sensor control device carrier nested with the trigger member according to an example of the present disclosure.
FIG. 10A is a front view of the sensor applicator according to an example of the present disclosure.
FIG. 10B is a cross-sectional view of the sensor applicator taken along the cutting plane AA' in FIG. 10A according to an example of the present disclosure.
FIG. 10C is a cross-sectional view of the sensor applicator according to an example of the present disclosure taken along a cutting plane perpendicular to the cutting plane AA'.
FIG. 11 is a schematic structural view of the housing according to an example of the present disclosure.
FIG. 12 is an exploded schematic view of the sealing assembly according to an example of the present disclosure.
FIG. 13 is an exploded schematic view of the sensor applicator cap according to an example of the present disclosure.

### Explanation of Reference Numerals

1: Instrument kit, 2: Reading device,
10: Sensor applicator, 20: Sensor control device, 30: Sensor applicator cap,
11: Housing, 12: Trigger member, 13: Sensor control device carrier, 14: Sharp module, 15: First driving member, 16: Second driving member, 21: Sensor, 22: Sensor cap, 23: Outer shell, 31: Drying member, 32: Decapping structure,
111: Second locking mechanism, 112: Third triggering mechanism, 113: Sixth guide rail, 121: First triggering mechanism, 122: Second triggering mechanism, 123: Second triggered mechanism, 124: Third main body part, 125: Fourth main body part, 130: Loading part, 131: First locking mechanism, 132: First triggered mechanism, 133: First limiting mechanism, 134: First through hole, 135: Activity chamber, 136: First main body part, 137: Second main body part, 138: Accommodation cavity, 139: Top cover, 310: Fourth through hole, 141: Fixing base, 142: Sharp,
1121: Second locking structure, 1122: Second sliding structure, 1231: First locking structure, 1232: First sliding structure, 1241: Third through hole, 1242: Fifth guide rail, 1243: Second vertical plate, 1244: Third vertical plate, 1251: Second through hole, 1252: Fourth guide rail, 1361: Blocking structure, 1362: First guide rail, 1363: Third guide rail, 1371: First vertical plate, 1401: Second guide rail, 1402: Inclined surface, 1403: Fixing structure, 1411: First retaining mechanism, 1412: Fixing part, 1413: Restricting part, 1414: Fixing cavity, 1421: Needle-shaped part, 1422: Cap part, 1423: Neck part, 1424: Clamping part.

### Detailed Description

Preferred embodiments of the present disclosure will be described in detail below with reference to the accompanying drawings. In the following description, the same components are denoted by the same reference numerals and redundant descriptions are omitted. Furthermore, the drawings are schematic diagrams, and the ratios between components or the shapes of the components, etc., may differ from the actual ones.

It should be noted that the terms "include" and "have" and any variations thereof in this disclosure, such as a process, method, system, product or device that includes or has a series of steps or units, are not necessarily limited to those steps or units clearly listed, but may include or have other steps or units that are not clearly listed or are inherent to these processes, methods, products or devices.

It should be noted that, herein, relative position and orientation terms such as "up", "upward", "down", "downward", "up-down direction", "left side", "leftward", "left", "towards the left", "right side", "rightward", "right", "towards the right", "left-right direction", "front", "forward", "rear", "rearward", and "front-rear direction" are used with reference to the normal operating postures and should not be considered as restrictive.

The present disclosure relates to a sensor applicator that may be configured to apply a sensor control device to a host, for instance, to affix the sensor control device to the host's body surface, or to place the sensor control device fully or partially subcutaneously within the host. The sensor applicator described herein can facilitate the application of the sensor control device to a desired location.

The sensor applicator described in the present disclosure may also be referred to as, for example, a booster device, a delivery device, an implantation device, an application device, or an auxiliary device. Similarly, the sensor control device may also be referred to as a sensor assembly, a medical device, or a detection device. It should be noted that these various terms are used to denote the apparatus for applying the sensor control device to a host as described herein, and should not be construed as limiting.

Hereinafter, the sensor applicator, the sensor control device, and the instrument kit comprising said sensor applicator and sensor control device according to the present disclosure will be described in detail with reference to the accompanying drawings.

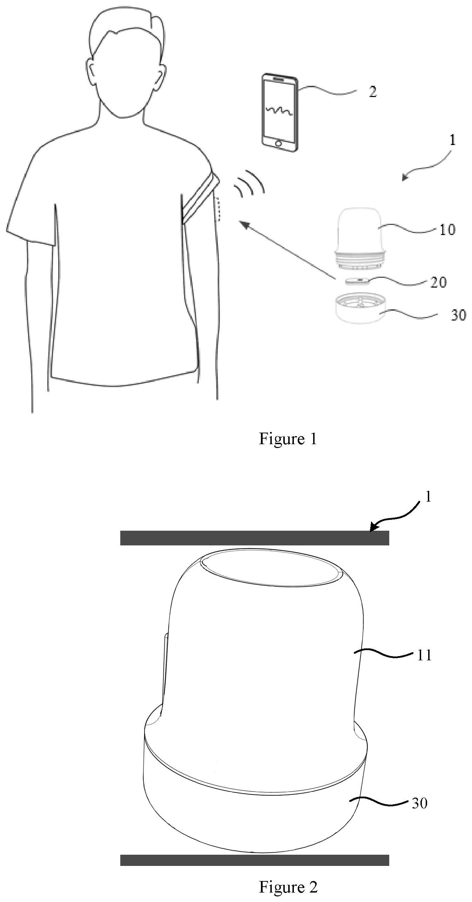

Figure 1 is a schematic diagram illustrating an application scenario of the sensor applicator 10 according to an example of this disclosure. In this disclosure, the sensor control device 20 can be applied to the host and to a desired location via the sensor applicator 10. The host can acquire physiological information through the sensor control device 20 applied to the host.

Furthermore, the present disclosure provides a monitoring system, which may comprise a sensor control device 20 configured to acquire physiological information of a host, a sensor applicator 10 configured to apply the sensor control device 20 to the host, and a reading device 2 configured to communicate with the sensor control device 20 (see FIG. 1). The sensor control device 20 applied to the host can transmit the acquired physiological information (e.g., wirelessly) to the reading device 2, thereby allowing the host to read and monitor their physiological information.

Furthermore, in some embodiments, the present disclosure may also provide a sensor applicator cap 30 (see FIG. 1). In some embodiments, the sensor applicator cap 30 may be connected to the sensor applicator 10 to fully or partially accommodate the sensor control device 20 described herein. In some embodiments, before being applied to the host, the sensor control device 20 may be fully or partially accommodated within the sensor applicator 10. In some embodiments, the connection method between the sensor applicator cap 30 and the sensor applicator 10 may be an easily detachable connection method such as a screw connection, an engagement connection, etc.

In addition, the present disclosure further provides an instrument kit 1. The instrument kit 1 may comprise a sensor control device 20 configured to acquire physiological information of a host, and a sensor applicator 10 configured to apply the sensor control device 20 to the host according to the present disclosure. Before being applied to a host, the sensor control device 20 can be fully accommodated in the sealed space formed by the connection between the sensor applicator cap 30 and the sensor applicator 10. When the sensor control device 20 is required for use, the sensor applicator cap 30 is removed, thereby triggering the sensor applicator 10 to apply the sensor control device 20 to the host. In this configuration, the sensor control device 20 is pre-packaged within the sensor applicator 10, and the instrument kit 1 is usable after removing the sensor applicator cap 30. In other words, it is possible to directly operate the sensor applicator 10 to apply the sensor control device 20 to the host. Compared to the prior art techniques that require additionally retrieving the sensor applicator 10 from another packaging component which packages the sensor applicator 10, the configuration is capable of simplifying the operator's operational steps and improving the operator's operational experience.

In some embodiments, the sensor control device 20 may be an analyte sensor device. The sensor in the sensor control device 20 may be an analyte sensor, which can generate information of a specific analyte in a bodily fluid based on the bodily fluid, for example, which can react with the analyte in the bodily fluid and generate analyte information. In this configuration, the sensor's reaction with the analyte in the bodily fluid facilitates the acquisition of analyte information in the bodily fluid.

In the present disclosure, the analyte targeted by the analyte sensor device may be, one or more of the following: glucose, acetylcholine, amylase, bilirubin, cholesterol, chorionic gonadotropin, creatine kinase, creatine, creatinine, DNA, fructosamine, glutamine, growth hormone, hormones, ketone bodies, lactate, oxygen, peroxide, prostate-specific antigen, prothrombin, RNA, thyroid-stimulating hormone, or troponin.

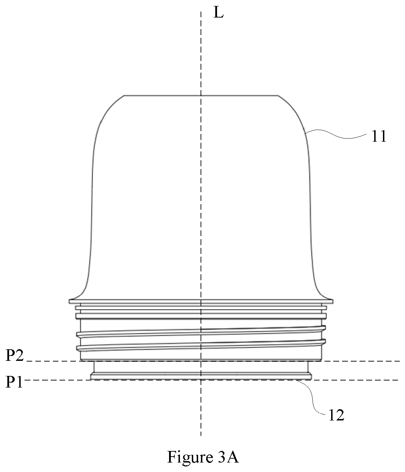

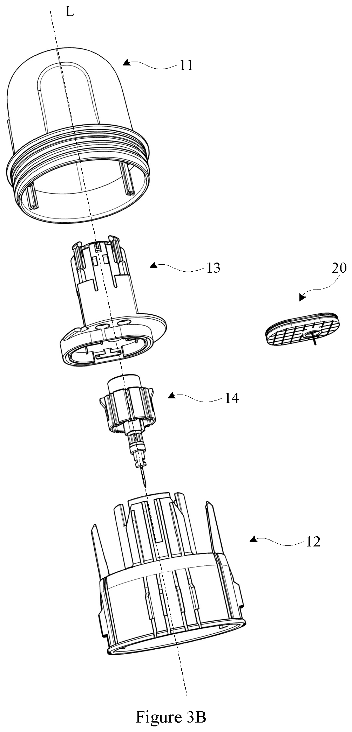

FIG. 2 is a schematic external view of the instrument kit 1 according to an example of the present disclosure. FIG. 3A is a schematic external view of the sensor applicator 10 loaded with the sensor control device 20 according to an example of the present disclosure. FIG. 3B is an exploded schematic view of FIG. 3A. Herein, FIG. 3A may represent the sensor applicator 10 in its initial state, which refers to the state before any operation has been performed on the sensor applicator 10 or its internal components. The initial state of the sensor applicator 10 may be an initial state in which the sensor applicator 10 comprises its various components (including the housing 11, trigger member 12, sensor control device carrier 13, sharp module 14, etc.).

Referring to FIG. 2, in some embodiments, the assembled product comprising sensor applicator 10, sensor control device 20, and sensor applicator cap 30 presents an appearance as shown in FIG. 2.

In some embodiments, the sensor applicator 10 may include housing 11.

In some embodiments, the housing 11 of the sensor applicator 10 can be assembled with the sensor applicator cap 30 to form the instrument kit 1 in a sealed packaged state. Thereby, protection for the internal sensor control device 20 and other components can be provided.

In some embodiments, the structures of the housing 11 and the sensor applicator cap 30 may be designed into shapes that facilitate gripping or holding by an operator. Consequently, the operator can easily carry the instrument kit 1 or conveniently perform operations on it.

In some embodiments, the appearance of the sensor applicator 10 or the instrument kit 1 may have a precisely or substantially bilaterally symmetrical design about axis L. This enhances the overall aesthetics of the sensor applicator 10 or the instrument kit 1.

Referring to FIG. 3A, in some embodiments, the sensor applicator 10 may include housing 11 and trigger member 12.

In some embodiments, when using the sensor applicator 10, the trigger member 12 may conform to the host's skin. The host's skin can thereby serve as a support surface for the trigger member 12. Subsequently, the operator can directly apply an action to the housing 11 (e.g., pressing the housing 11), thereby causing the sensor control device 20 inside the sensor applicator 10 to be applied to the host.

In some embodiments, the trigger member 12 may extend beyond the housing 11 and be in a first position P1 in the initial state, and the housing may be in a second position P2 in the initial state. In some embodiments, the housing 11 may trigger the trigger member 12 by moving from the second position P2 to the first position P1. The distance between the first position P1 and the second position P2, or in other words, the distance the housing 11 moves, is designed to be sufficient to trigger the movement of the various internal components of the sensor applicator 10. This distance should not be too short, otherwise it will be difficult to achieve the effect of triggering the movement of the internal components of the sensor applicator 10; of course, it should not be too long either, otherwise it will affect the operator's operating experience.

One objective of the present disclosure is to provide a sensor applicator 10 capable of improving triggering effectiveness and operator experience.

Referring to FIG. 3B, the present disclosure provides a sensor applicator 10. In some embodiments, the sensor applicator 10 may be configured to apply the sensor control device 20 to a host.

In some embodiments, the sensor applicator 10 may have a proximal end and a distal end and include a housing 11, a sensor control device carrier 13, a sharp module 14, and a trigger member 12. The sensor control device carrier 13 may be configured to carry the sensor control device 20. The sharp module 14 may assist in applying the sensor control device 20 to a host. The trigger member 12 may be configured, when triggered, to cause the sensor control device carrier 13 and the sharp module 14 to move the sensor control device 20 from the distal end to the proximal end. The proximal end may be an end close to the host, and the distal end may be an end away from the host.

In some embodiments, the sensor control device carrier 13 may be configured to releasably receive the sensor control device 20. In this configuration, the sensor control device carrier 13 can be configured to accommodate the sensor control device 20 before releasing the sensor control device 20, and can be configured to apply the sensor control device 20 to the host after releasing the sensor control device 20.

In some embodiments, the sharp module 14 may be configured to releasably apply the sensor control device 20 to the host. In some embodiments, the sharp module 14 may possess properties facilitating easy skin insertion. In this configuration, by leveraging the sharp module 14's property of easy skin insertion, the sensor control device 20 can be effectively applied to the host. Furthermore, the sharp module 14 can detach from the sensor control device 20 after application to the host.

In some embodiments, the trigger member 12 may be configured such that, when triggered by the housing 11, it causes the sensor control device carrier 13 to drive the sharp module 14 and the sensor control device 20 to move from the distal end to the proximal end.

In some embodiments, the sharp module 14 is further configured to be triggered by the trigger member 12 when it moves a predetermined distance relative to the trigger member 12 under the drive of the sensor control device carrier 13, and to move from the proximal end to the distal end. In this configuration, when the sharp module 14 moves a predetermined distance relative to the trigger member, or after completing the action of applying the sensor control device 20 to the host, the sharp module 14 is triggered by the trigger member to retract to the distal end, which can reduce the damage of the sharp module 14 to the host.

In the sensor applicator 10 of the present disclosure, when the trigger member is triggered by the housing, it causes the sensor control device carrier 13 to drive the sharp module 14 and the sensor control device 20 to move from the distal end away from the host to the proximal end close to the host. This configuration allows an operator to indirectly trigger the trigger member 12 by triggering the housing 11, thereby applying the sensor control device 20 to the host. In this configuration, since the housing 11 provides a larger force application area compared to the trigger button in the prior art, the operator can apply force to the housing 11 more effectively by triggering the housing 11 and the trigger member 12 in a coordinated manner. This can improve the effectiveness of the trigger and the operator's operating experience.

In some embodiments, during use of the sensor applicator 10, the trigger member 12 may contact the host's skin. In this configuration, the support force provided by the host's skin enables more effective triggering of the trigger member 12.

In some embodiments, the housing 11 may be movably connected to the trigger member 12. In some embodiments, the trigger member 12 may be movably connected to the sensor control device carrier 13. In some embodiments, the sensor control device carrier 13 may be movably connected to the sharp module 14. In some embodiments, the sensor control device carrier 13 may be detachably connected to the sensor control device 20. In this configuration, movement of the housing 11 can trigger movement of the trigger member 12; movement of the trigger member 12 can trigger movement of the sensor control device carrier 13; and movement of the sensor control device carrier 13 can drive movement of the sharp module 14 and the sensor control device 20. Thereby, the complete operational process of applying the sensor control device 20 to the host can be achieved.

In some embodiments, the housing 11, trigger member 12, sensor control device carrier 13, and sharp module 14 may have a precisely or substantially axisymmetric design about axis L. This configuration can reduce the complexity of the sensor applicator 10, enhance its structural stability, and also facilitate the machining and molding of the various components (including the housing 11, trigger member 12, sensor control device carrier 13, and sharp module 14).

In some embodiments, the housing 11 may be an integrated structure. In some embodiments, the trigger member 12 may be an integrated structure. In some embodiments, the sensor control device carrier 13 may be an integrated structure. This enhances the strength, stiffness, and stability of the components (housing 11, trigger member 12, or sensor control device carrier 13), while simultaneously simplifying the manufacturing process and reducing assembly time.

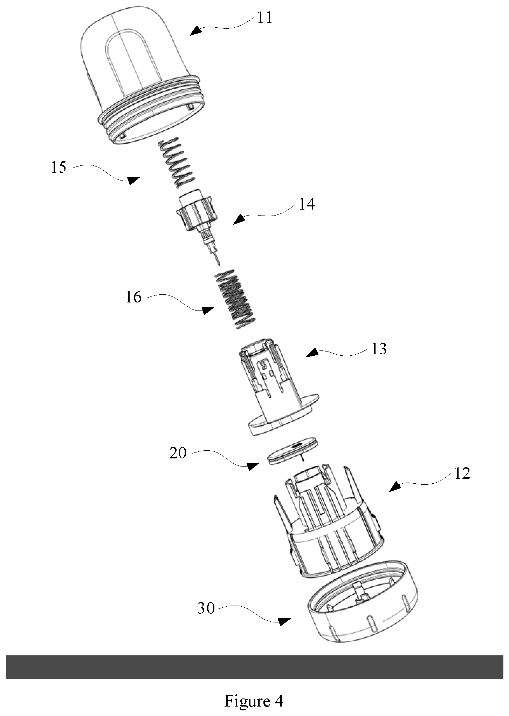

FIG. 4 is an exploded schematic view of the instrument kit 1 according to an example of the present disclosure.

Referring to FIG. 4, in some embodiments, the sensor applicator 10 may include a first driving member 15.

In some embodiments, the first driving member 15 may be configured to drive the sensor control device carrier 13 to move from the distal end to the proximal end when the trigger member 12 is triggered by the housing 11. In this configuration, driven by the first driving member 15, the sensor control device carrier 13 causes the sharp module 14 and the sensor control device 20 to move from the distal end to the proximal end, thereby enabling application of the sensor control device 20 to the host.

In some embodiments, the first driving member 15 may be an elastic element. The elastic element may be, for example, a spring.

In some embodiments, the first driving member 15 may be sleeved over the sensor control device carrier 13 and abut on the top of the housing 11.

In some embodiments, the first driving member 15 may be disposed between the sensor control device carrier 13 and the housing 11. In some embodiments, in the initial state, the first driving member 15 may be in a compressed state. In this configuration, when the trigger member 12 is triggered by the housing 11, the stored elastic potential energy in the first driving member 15 is released, thereby driving the sensor control device carrier 13 to move from the distal end to the proximal end.

Referring to FIG. 4, in some embodiments, the sensor applicator 10 may include a second driving member 16.

In some embodiments, the second driving member 16 may be configured to drive the sharp module 14 to move from the proximal end to the distal end when the sharp module 14 has moved a predetermined distance relative to the trigger member 12 and is triggered by the trigger member 12. In this configuration, driven by the second driving member 16, the sharp module 14 can retract to the distal end after applying the sensor control device 20 to the host.

In some embodiments, the second driving member 16 may be an elastic element. The elastic element may be, for example, a spring.

In some embodiments, the second driving member 16 may be disposed between the sharp module 14 and the sensor control device carrier 13. In some embodiments, in the initial state, the second driving member 16 may be in a compressed state. In this configuration, when the sharp module 14 has moved the predetermined distance relative to the trigger member 12 and is triggered by the trigger member 12, the stored elastic potential energy in the second driving member 16 is released, thereby driving the sharp module 14 to retract to the distal end after applying the sensor control device 20 to the host.

In some embodiments, the trigger member 12, sensor control device carrier 13, sharp module 14, first driving member 15, second driving member 16, and sensor control device 20 may be assembled within the sealed package formed by the housing 11 and the sensor applicator cap 30.

In some embodiments, the trigger member 12, sensor control device carrier 13, sharp module 14, first driving member 15, and second driving member 16 may have a precisely or substantially axisymmetric structure about axis L. This enhances the internal structural stability of the instrument kit 1. Hereinafter, axis L may be referred to as the axis L of the trigger member 12, sensor control device carrier 13, sharp module 14, first driving member 15, and second driving member 16, respectively.

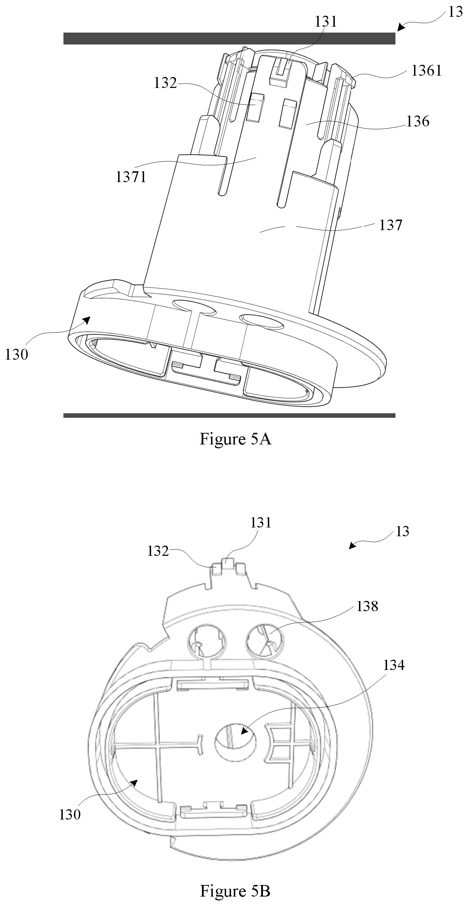

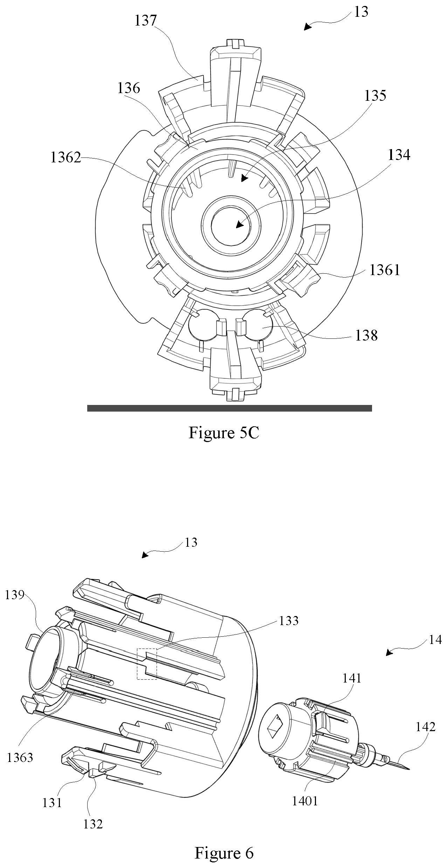

FIG. 5A is a schematic perspective view of the sensor control device carrier 13 according to an example of the present disclosure from one viewing angle. FIG. 5B is a schematic bottom view of FIG. 5A. FIG. 5C is a schematic top view of FIG. 5A.

Referring to FIGS. 5A to 5C, in some embodiments, the sensor control device carrier 13 may include a first locking mechanism 131. In some embodiments, the sensor control device carrier 13 may include a first triggered mechanism 132.

In some embodiments, the first locking mechanism 131 may be configured to be locked with the housing 11 in the initial state. In some embodiments, the first triggered mechanism 132 may be configured to, when triggered by the trigger member 12, cause the sensor control device carrier 13 to move from the distal end to the proximal end. In this configuration, the first locking mechanism 131 can lock the sensor control device carrier 13 to the housing 11 in the initial state, which can effectively reduce the possibility of the sensor control device carrier 13 being accidentally triggered; the first triggered mechanism 132 enables the sensor control device carrier 13 to release the locked state between the first locking mechanism 131 and the housing 11 when triggered by the triggered element 12, thereby enabling the sensor control device carrier 13 to move from the distal end to the proximal end.

In some embodiments, the sensor control device carrier 13 may have a loading part 130. The loading part 130 may be used for loading the sensor control device 20.

In some embodiments, a first through hole 134 may be formed at the location of the axis L of the sensor control device carrier 13. The first through hole 134 may allow a portion of the sharp module 14 to pass through.

In some embodiments, the sharp module 14 may include a needle-shaped part. In some embodiments, the first through hole 134 may allow the needle-shaped part of the sharp module 14 to pass through.

In some embodiments, an activity chamber 135 may be formed at the location of the axis L of the sensor control device carrier 13. The sharp module 14 may move within the activity chamber 135. For example, when the sharp module 14 moves from the proximal end toward the distal end, the activity chamber 135 may provide a movement channel in which the sharp module 14 moves from the proximal end toward the distal end.

In some embodiments, the sensor control device carrier 13 may include a first main body part 136. The first main body part 136 may be configured to form the activity chamber 135.

In some embodiments, an end of the first main body part 136 toward the distal end may have a blocking structure 1361. The blocking structure 1361 may be configured to contact the trigger member 12 when the sensor control device carrier 13 has moved toward the proximal end to a certain distance, thereby limiting the continuing movement of the sensor control device carrier 13 toward the proximal end.

Referring to FIG. 5C, in some embodiments, there may be a plurality of blocking structures 1361, for example, 2, 3, 4, etc. In some embodiments, the blocking structures 1361 may be uniformly disposed around the top of the first main body part 136.

In some embodiments, the first main body part 136 may pass through the trigger member 12. In some embodiments, the sensor control device carrier 13 and the trigger member 12 may be arranged in a nested configuration. This arrangement saves internal space within the instrument kit 1.

In some embodiments, the sensor control device carrier 13 may include a second main body part 137. In some embodiments, the second main body part 137 may form an accommodation cavity 138.

In some embodiments, at least a portion of the accommodation cavity 138 may be configured to accommodate a magnetic component. The magnetic component may function as a control switch for the sensor control device 20.

In some embodiments, the magnetic component may be fixed within the accommodation cavity 138.

In some embodiments, when the magnetic component is proximate to the sensor control device 20, the sensor control device 20 is in an inactive or dormant state; when the magnetic component is distant from the sensor control device 20, the sensor control device 20 is in an active state. In this configuration, when the sensor control device 20 is applied to the host and detaches from the loading part 130 of the sensor control device carrier 13, the magnetic component becomes distant from the sensor control device 20. Consequently, the sensor control device 20 enters its active state upon application to the host, enabling it to properly monitor the host's physiological status.

In some embodiments, the second main body part 137 may pass through the trigger member 12. In some embodiments, the sensor control device carrier 13 and the trigger member 12 may be arranged in a nested configuration. This arrangement saves internal space within the instrument kit 1 and facilitates contact-based triggering of the sensor control device carrier 13 by the trigger member 12.

In some embodiments, the second main body part 137 may be disposed outside the first main body part 136.

In some embodiments, the first driving member 15 may be sleeved over the first main body part 136 and disposed on the second main body portion 137. In other words, the second main body part 137 may serve to support the first driving member 15.

Referring to FIG. 5A, in some embodiments, the first triggered mechanism 132 and the first locking mechanism 131 may be disposed at a position of the second main body part 137 close to the distal end. In some embodiments, the first triggered mechanism 132 and the first locking mechanism 131 may be adjacently arranged. In some embodiments, the first locking mechanism 131 may be positioned closer to the distal end compared to the first triggered mechanism 132 (refer to FIG. 5A, the first locking mechanism 131 may be positioned above the first triggered mechanism 132).

In some embodiments, the second main body part 137 may have a first vertical plate 1371 extending toward the distal end. In some embodiments, the first triggered mechanism 132 and the first locking mechanism 131 may be disposed at a side of the first vertical plate 1371 close to the distal end.

In some embodiments, the first vertical plate 1371 may possess a certain degree of elasticity. When the first triggered mechanism 132 is triggered, the first vertical plate 1371 can drive the first triggered mechanism 132 and the first locking mechanism 131 to shift toward the first main body 136, thereby unlocking the first locking mechanism 131.

In some embodiments, there may be two second main body parts 137. The two second main body parts 137 may be symmetrically disposed at both sides of the first main body part 136 along axis L (refer to FIG. 3B). This configuration enhances the structural stability of the sensor control device carrier 13 and makes the sensor control device carrier 13 and housing 11 more securely locked.

FIG. 6 is a schematic perspective view of the sensor control device carrier 13 and the sharp module 14 according to an example of the present disclosure.

Referring to FIG. 6, in some embodiments, the sensor control device carrier 13 may include a first limiting mechanism 133.

In some embodiments, the sharp module 14 may include a first retaining mechanism 1411.

In some embodiments, the first limiting mechanism 133 may be configured to restrict movement of the sharp module 14 relative to the sensor control device carrier 13 by engaging with the first retaining mechanism 1411. In this configuration, the first retaining mechanism 1411 of the sharp module 14 can be engaged with the first limiting mechanism 133 of the sensor control device carrier 13, maintaining the sharp module 14 and the sensor control device carrier 13 in a relatively stationary state. Consequently, the sensor control device carrier 13 can drive the sharp module 14 to move together.

In some embodiments, there may be a plurality of first limiting mechanisms 133 and, correspondingly, a plurality of first retaining mechanisms 1411. The plurality of first limiting mechanisms 133 may be uniformly arranged on the first main body part 136, and the plurality of first retaining mechanisms 1411 may be uniformly arranged at the top of the sharp module 14.

Referring to FIGS. 4 and 6, in some embodiments, the first retaining mechanism 1411 may be an outwardly protruding protrusion, and the first limiting mechanism 133 may be a baffle configured to block movement of the protrusion toward the distal end. In some embodiments, in the initial state, the second driving member 16 may be compressed between the sharp module 14 and the bottom of the first main body part 136. The upward driving force from the second driving member 16 in the initial state causes the first retaining mechanism 1411 to abut against the first limiting mechanism 133 upwardly. Consequently, when the sensor control device carrier 13 moves downward, it can drive the sharp module 14 to move together toward the proximal end until the first retaining mechanism 1411 is triggered by the trigger member 12.

In some embodiments, the sharp module 14 may include a needle-shaped part 1421. The needle-shaped part 1421 may be connected to the sensor of the sensor control device 20.

In some embodiments, the sharp module 14 may be at least partially disposed within the activity chamber 135 formed by the first main body part 136. In some embodiments, the needle-shaped part 1421 may be exposed from the activity chamber 135 and extend toward the proximal end. In this configuration, the needle-shaped part 1421's property of easy skin insertion is utilized to drive and implant the sensor into the host.

Referring to FIG. 6, in some embodiments, the applicator 10 may include a top cover 139. In some embodiments, the top cover 139 may be disposed at the top of the sensor control device carrier 13. In some embodiments, the top cover 139 may be fixed to the top of the sensor control device carrier 13. In this configuration, the top cover 139 serves to block movement of the sharp module 14 within the activity chamber 135. For example, during the process where the sharp module 14 is driven by the second driving member 16 to move from the proximal end to the distal end, the top cover 139 can prevent the sharp module 14 from ejecting out of the activity chamber 135.

Referring to FIGS. 5C and 6, in some embodiments, a plurality of first guide rails 1362 may be provided on the inner wall of the first main body part 136, and a plurality of second guide rails 1401 may be provided on the outer surface of the sharp module 14 at positions corresponding to the first guide rails 1362. In this configuration, by the matchment of the plurality of first guide rails 1362 and the plurality of second guide rails 1401, it enables the sharp module 14 to move stably within the first main body part 136 along a path defined by the first guide rails 1362, including movement from the distal end to the proximal end or from the proximal end to the distal end. This ensures that the sharp module 14 applies the sensor control device 20 to the host with greater stability.

In some embodiments, the number of first guide rails 1362 may match the number of second guide rails 1401. In some embodiments, the first guide rails 1362 may be uniformly arranged on the inner wall of the first main body part 136, and the second guide rails 1401 may be uniformly arranged on the outer surface of the sharp module 14. Consequently, the sharp module 14 moves more stably within the first main body part 136, enhancing the stability of the force applied by the sharp module 14 to the sensor control device 20.

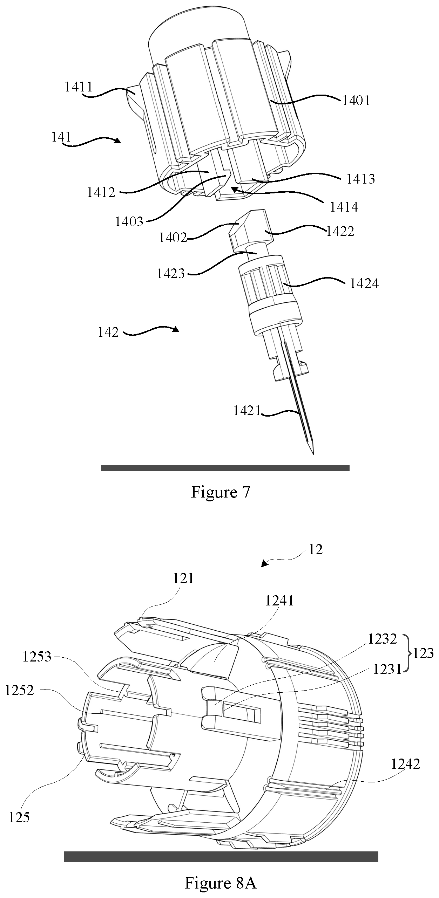

FIG. 7 is an exploded schematic view of the sharp module 14 according to an example of the present disclosure.

Referring to FIG. 7, in some embodiments, the sharp module 14 may include a fixing base 141 and a sharp 142. The fixing base 141 may be configured to fix or retain the sharp 142.

In some embodiments, the first retaining mechanism 1411 may be disposed at an end of the fixing base 141 close to the distal end. When the fixing base 141 is engaged onto the sensor control device carrier 13 via the first retaining mechanism 1411, the sensor control device carrier 13 can drive the sharp module 14 to move together toward the proximal end.

In some embodiments, the fixing base 141 may include a fixing part 1412. In some embodiments, the fixing part 1412 may be configured to be deflectable. In some embodiments, the fixing part 1412 may possess a certain degree of elasticity.

In some embodiments, the fixing part 1412 may have a fixing structure 1403 for retaining the sharp 142. In some embodiments, the fixing structure 1403 may be a hook-shaped or protruding portion of the fixing part 1412.

In some embodiments, the fixing base 141 may include a restricting part 1413. The restricting part 1413 may be configured to restrict movement or rotation of the sharp 142 within the fixing base 141.

In some embodiments, the fixing base 141 may include a fixing cavity 1414. The fixing cavity 1414 may be configured to accommodate and fix the sharp 142. In some embodiments, the fixing part 1412 and the restricting part 1413 may cooperate to form the fixing cavity 1414.

In some embodiments, the sharp 142 may at least partially accommodate the sensor.

In some embodiments, the sharp 142 may include a needle-shaped part 1421.

In some embodiments, the needle-shaped part 1421 may have a groove extending along its length direction (which may be simply referred to as a needle groove). The sensor may be at least partially disposed within the needle groove of the sharp 142. In this configuration, the sensor is received in the needle groove, thereby being positioned subcutaneously in the host following the motion of the sharp 142.

In some embodiments, the sharp 142 may include a cap part 1422. The cap part 1422 may be used for engagement with the fixing base 141.

In some embodiments, the sharp 142 may include a neck part 1423. The neck part 1423 may cooperate with the cap part 1422 to engage with both the restricting part 1413 and the fixing part 1412 of the fixing base 141.

In some embodiments, the neck part 1423 may be connected to the cap part 1422. In some embodiments, a radial dimension of the neck part 1423 may be smaller than a radial dimension of the cap part 1422. Thereby, an annular recess located outside the neck part 1423 can be formed in the sharp 142.

In some embodiments, when projecting along the central axis of the sharp 142, if the resulting projection is circular, the radial dimension may be the diameter of the circle; if the projection is polygonal or irregular, the radial dimension may be the length of the longest diagonal of the polygons or irregular shapes. In some embodiments, the central axis of the sharp 142 may coincide with axis L.

In some embodiments, the sharp 142 may include a clamping part 1424. The clamping part 1424 may be configured to clamp the needle-shaped part 1421.

In some embodiments, the clamping part 1424 may be connected to the cap part 1422 via the neck part 1423. In some embodiments, a radial dimension of the clamping part 1424 may be greater than a radial dimension of the neck part 1423.

In some embodiments, when the fixing base 141 is engaged with the sensor control device carrier 13 via the first retaining mechanism 1411 and is driven to move toward the distal end of the sensor applicator 10, the sensor can be positioned subcutaneously in the host by the sharp 142 during the process of the sharp 142 piercing the host's skin. After subcutaneous placement of the sensor, the first retaining mechanism 1411 of the fixing base 141 is triggered by the trigger member 12, disengaging it from the sensor control device carrier 13. The fixing base 141 can then move toward the proximal end of the sensor applicator 10, and the sharp 142 is retracted from the host subcutaneously, driven by the fixing base 141 (this process may be referred to as retraction of the sharp 142).

In some embodiments, a hollow structure or a recessed structure that is recessed inward from the outer surface of the clamping part 1424 can be provided in the clamping part 1424. This reduces the total weight of the sharp 142, which in turn facilitates the retraction of the sharp 142 driven by the fixing base 141.

In some embodiments, the fixing cavity 1414 may be configured to accommodate and fix the cap part 1422 of the sharp 142.

In some embodiments, the restricting part 1413 may be configured to restrict movement or rotation of the cap part 1422 within the fixing base 141.

In some embodiments, the cap part 1422 may have an inclined surface 1402. In one embodiment, the fixing structure 1403 may have a inclined surface corresponding to the inclined surface 1402. The cap part 1422 can slide into the fixing cavity 1414 of the fixing base 141 via the inclined surface 1402. During the process of the cap part 1422 sliding into the fixing base 141, it first causes the fixing part 1412 to deflect toward one side. When the cap part 1422 has slid into the fixing cavity 1414, the fixing structure 1403 of the fixing part 1412 returns to its original position, engaging with the bottom surface of the cap part 1422 adjacent to the neck part 1423. This configuration allows the sharp 142 to be securely engaged within the fixing base 141, enabling it to follow the fixing base 141 in stable motion.

In some embodiments, during the assembly of the sharp 142 with the fixing base 141, the top surface of the cap part 1422 contacts the retaining structure 111, causing the fixing part 1412 to deflect away from the central axis of the fixing base 141. This facilitates the assembly of the sharp 142 with the fixing base 141.

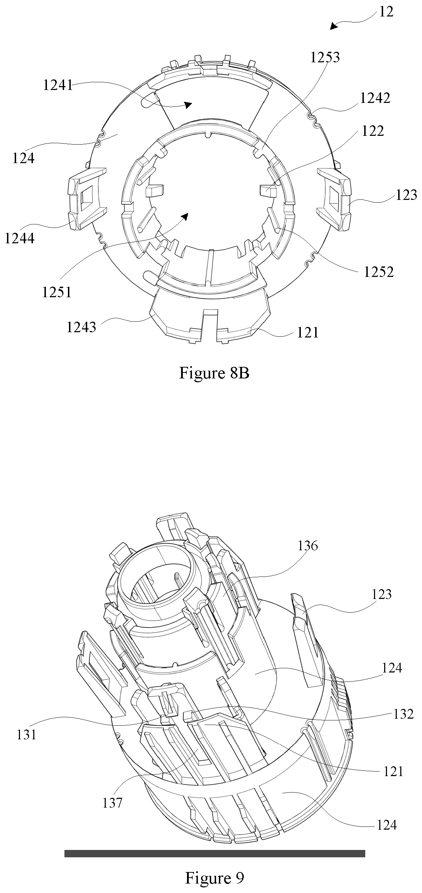

FIG. 8A is a schematic perspective view of the trigger member 12 according to an example of the present disclosure, shown from one viewing angle. FIG. 8B is a schematic perspective view of the trigger member 12 according to an example of the present disclosure, shown from another viewing angle. FIG. 9 is a schematic structural view showing the sensor control device carrier 13 nested with the trigger member 12 according to an example of the present disclosure.

Referring to FIGS. 8A to 9, in some embodiments, the trigger member 12 may include a first triggering mechanism 121. In some embodiments, the first triggering mechanism 121 can be configured to trigger the first triggered mechanism 132 when the trigger member 12 is triggered by the housing 11. In this configuration, triggering the first triggered mechanism 132 of the sensor control device carrier 13 by the first triggering mechanism 121 of the trigger member 12 releases the locking between the sensor control device carrier 13 and the housing 11, thereby allowing the sensor control device carrier 13 to move from the distal end to the proximal end.

In some embodiments, referring to FIGS. 6 and 8B, the trigger member 12 may include a second triggering mechanism 122. In some embodiments, the second triggering mechanism 122 may be configured to trigger the first retaining mechanism 1411 when the sharp module 14 has moved a predetermined distance. In this configuration, when the sharp module 14 has moved the predetermined distance relative to the trigger member 12 and contacts the second triggering mechanism 122 of the trigger member 12, it is triggered by the second triggering mechanism 122, thereby disengaging from the restriction of the sensor control device carrier 13 and moving from the proximal end to the distal end. This allows the sharp module 14 to retract to the distal end following the application of the sensor control device 20 to the host.

In some embodiments, the predetermined distance may be the distance between the first retaining mechanism 1411 and the second triggering mechanism 122 in the initial state. The predetermined distance can be determined based on experimental values, such as based on the distance that the sensor control device carrier 13 needs to move when the sharp module 14 applies the sensor control device 20 to the host, that is, the distance that the sensor control device carrier 13 needs to move, that is, the distance between the first retaining mechanism 1411 of the sharp module 14 and the second triggering mechanism 122 of the trigger member 12.

In some embodiments, the first retaining mechanism 1411 may possess a certain degree of elasticity. When the first retaining mechanism 1411 moves towards the proximal end to contact the second triggering mechanism 122, the second triggering mechanism 122 causes the first retaining mechanism 1411 to deflect toward the internal space of the first main body part 136. Consequently, the first retaining mechanism 1411 disengages from the first limiting mechanism 133. Thus, the sharp module 14 can be moved from the proximal end to the distal end under the drive of the second driving member 16.

In some embodiments, the trigger member 12 may include a third main body part 124 and a fourth main body part 125. The fourth main body part 125 may be formed on the third main body part 124.

In some embodiments, the third main body part 124 and the fourth main body part 125 may cooperate to form a second through hole 1251. The second through hole 1251 may allow the first main body part 136 to pass through. In some embodiments, a third through hole 1241 may be formed at the top of the third main body part 124. The third through hole 1241 may allow the second main body part 137 to pass through. This configuration enables the nested arrangement of the sensor control device carrier 13 and the trigger member 12, saving internal space within the instrument kit 1.

In some embodiments, the first triggered mechanism 132 may possess a certain degree of elasticity. In some embodiments, the first vertical plate 1371 carrying the first triggered mechanism 132 may possess a certain degree of elasticity. This enables the first triggered mechanism 132 to move when triggered by the first triggering mechanism 121 on the trigger member 12.

Furthermore, in some embodiments, in the initial state, when the first main body part 136 passes through the second through hole 1251, the first triggered mechanism 132 provided on the first main body part 136 may be disposed close to or in contact with the first triggering mechanism 121. In this configuration, when the trigger member 12 moves toward the distal end, the first triggering mechanism 121 can trigger the first triggered mechanism 132 to move inward (towards the first main body part 136). Consequently, the first locking mechanism 131 is simultaneously moved inward and disengages from the housing 11, thereby allowing the sensor control device carrier 13 to move relative to the housing 11.

Referring to FIGS. 6 to 8B, in some embodiments, a third guide rail 1363 may be provided on the outer wall of the first main body part 136, and a fourth guide rail 1252 corresponding to the third guide rail 1363 may be provided on the inner wall of the fourth main body part 125. This configuration defines the movement path of the sensor control device carrier 13 within the trigger member 12, enabling more stable movement of both the sensor control device carrier 13 and the trigger member 12.

In some embodiments, the number of third guide rails 1363 and the number of fourth guide rails 1252 may be set correspondingly. In some embodiments, the third guide rails 1363 may be uniformly distributed on the outer wall of the first main body part 136. In some embodiments, the fourth guide rails 1252 may be uniformly distributed on the inner wall of the fourth main body part 125. This further enhances the movement stability of the sensor control device carrier 13 and the trigger member 12.

Referring to FIGS. 8A and 9, in some embodiments, the trigger member 12 may include a second triggered mechanism 123. In some embodiments, the second triggered mechanism 123 may be configured to be locked with the housing 11 in the initial state. The second triggered mechanism 123 may be configured to, when triggered by the housing 11, cause the first triggering mechanism 121 to trigger the first triggered mechanism 132. In this configuration, the trigger member 12 can be kept locked with the housing 11 via the second triggered mechanism 123 in the initial state. On the other hand, when the second triggered mechanism 123 is triggered by the housing 11, the first triggering mechanism 121 of the trigger member 12 is caused to trigger the first triggered mechanism 132 of the sensor control device carrier 13, thereby releasing the locked state between the first locking mechanism 131 of the sensor control device carrier 13 and the housing 11, and thus allowing the sensor control device carrier 13 to move from the proximal end to the distal end.

In some embodiments, referring to FIG. 8A, the second triggered mechanism 123 may include a first locking structure 1231 and a first sliding structure 1232. The first locking structure 1231 may be used for locking with the housing 11, and the first sliding structure 1232 may be used for releasing the locked state with the housing 11 when the trigger member 12 moves toward the distal end.

In some embodiments, the second triggered mechanism 123 may be disposed on the third main body part 124. In some embodiments, the first triggering mechanism 121 may be disposed on the third main body part 124. In some embodiments, there may be a plurality of first triggering mechanisms 121 and a plurality of second triggered mechanisms 123. For example, the number of first triggering mechanisms 121 and second triggered mechanisms 123 may each be two.

In some embodiments, the first triggering mechanisms 121 and the second triggered mechanisms 123 may be arranged around the periphery of the fourth main body part 125. For example, two first triggering mechanisms 121 may be located on the front and rear sides of the fourth main body part 125, and two second triggered mechanisms 123 may be located on the left and right sides of the fourth main body part 125. This configuration allows the first triggering mechanisms 121 and the second triggered mechanisms 123 to be arranged in a distributed manner on the third main body part 124, enabling the first triggering mechanisms 121 and the second triggered mechanisms 123 to perform their respective functions independently.

In some embodiments, the first triggered mechanism 132 may be a protrusion, and a surface of this protrusion corresponding to the first triggering mechanism 121 may be an inclined surface. This configuration enables the first triggering mechanism 121, when moving towards the distal end, to trigger the first triggered mechanism 132 to move inward, thereby driving the first locking mechanism 131 to move inward and consequently releasing the first locking mechanism 131 from its locked state with the housing.

In some embodiments, the first locking mechanism 131 may be a protrusion. The design of this protrusion should allow for stable engagement with the housing 11.

In some embodiments, referring to FIG. 8B, a second vertical plate 1243 and a third vertical plate 1244 may be formed at a side of the third main body part 124 close to the distal end.

In some embodiments, a side of the second vertical plate 1243 close to the distal end may be configured to form the first triggering mechanism 121.

In some embodiments, a side of the third vertical plate 1244 close to the distal end may be configured to form the second triggered mechanism 123.

In some embodiments, the second vertical plate 1243 and the third vertical plate 1244 may be disposed around the periphery of the fourth main body part 125. In some embodiments, the second vertical plate 1243 and the third vertical plate 1244 may be arranged surrounding the fourth main body part 125.

In some embodiments, there may be two second vertical plates 1243, which are distributed on the front and rear sides of the fourth main body part 125. In some embodiments, there may be two third vertical plates 1244, which are distributed on the left and right sides of the fourth main body part 125.

In some embodiments, when the second main body part 137 passes through the third through hole 1241, the second vertical plate 1243 may be located outside the first vertical plate 1371, with the first triggering mechanism 121 on the second vertical plate 1243 disposed adjacent to the first triggered mechanism 132 on the first vertical plate 1371.

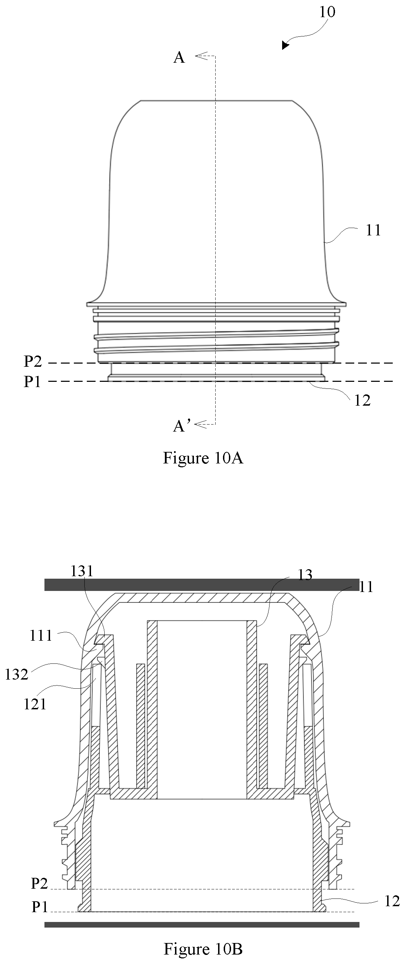

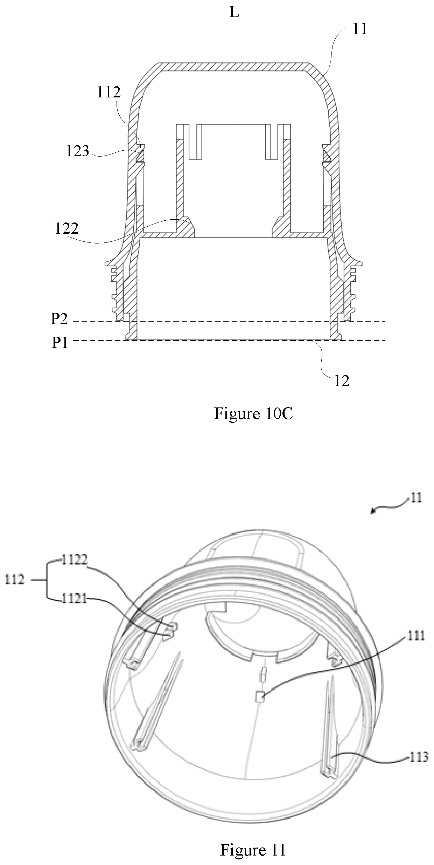

FIG. 10A is a front view of the sensor applicator 10 according to an example of the present disclosure. FIG. 10B is a cross-sectional view of the sensor applicator 10 taken along cutting plane AA' in FIG. 10A according to an example of the present disclosure. FIG. 10C is a cross-sectional view of the sensor applicator 10 taken along a cutting plane perpendicular to cutting plane AA' according to an example of the present disclosure. It should be noted that, to illustrate the internal structure of the sensor applicator 10 more clearly, FIGS. 10B and 10C omit some non-essential components, which should not be construed as limiting the present disclosure in any way. FIG. 11 is a schematic structural view of the housing 10 according to an example of the present disclosure.

Referring to FIGS. 10A to 10C, in some embodiments, the trigger member 12 may extend beyond the housing 11 and be in a first position P1 in the initial state, and the housing 11 may be in a second position P2 in the initial state. The housing 11 may trigger the trigger member 12 by moving from the second position P2 to the first position P1. In this configuration, moving the housing 11 relative to the trigger member 12 triggers the trigger member 12, thereby causing the sensor control device carrier 13 to drive the sharp module 14 and the sensor control device 20 to move from the distal end to the proximal end. This facilitates the operator's application of force to the housing 11 to apply the sensor control device 20 to the host, improving triggering effectiveness and the operator experience.

In some embodiments, a method of using the sensor applicator 10 may comprise: placing the trigger member 12 against the host's skin surface, for example, against the host's arm; utilizing the support force provided by the host, pressing the housing 11 towards the proximal end to move it from the second position P2 to the first position P1 (of course, FIGS. 10A to 10C are only schematic; the housing 11 need not strictly stop exactly at the first position P1, and being near the first position P1 is also acceptable); unlocking the sensor control device carrier 13 and causing it to move towards the proximal end, whereupon the sensor control device carrier 13 drives the sharp module 14 and the sensor control device 20 to move from the distal end to the proximal end. In this configuration, compared to prior art techniques that require a button on the housing, applying force directly to the housing 11 increases the force application area, enabling more effective triggering of the sensor control device carrier 13 to move towards the proximal end.

Referring to FIGS. 10A to 11, in some embodiments, the housing 11 may include a second locking mechanism 111. In some embodiments, the housing 11 may include a third triggering mechanism 112. In some embodiments, the second locking mechanism 111 may be configured to be locked with the first locking mechanism 131 in the initial state. In some embodiments, the third triggering mechanism 112 may be configured to be locked with the second triggered mechanism 123 in the initial state. In some embodiments, the third triggering mechanism 112 may be configured to trigger the second triggered mechanism 123 when the housing 11 moves relative to the trigger member 12. In this configuration, via the second locking mechanism 111, the housing 11 can be locked with the first locking mechanism 131 of the sensor control device carrier 13 in the initial state, reducing the possibility of accidental triggering of the sensor control device carrier 13 in the initial state. Via the third triggering mechanism 112, the housing 11 can be locked with the second triggered mechanism 123 of the trigger member 12 in the initial state, reducing the possibility of accidental triggering of the trigger member 12 in the initial state. Furthermore, when the housing 11 moves relative to the trigger member 12 (e.g., when the housing 11 is pressed), the third triggering mechanism 112 on the housing 11 triggers the second triggered mechanism 123 on the trigger member 12, thereby causing the first triggering mechanism 121 of the trigger member 12 to trigger the first triggered mechanism 132 of the sensor control device carrier 13, which in turn releases the locked state between the first locking mechanism 131 of the sensor control device carrier 13 and the housing 11, thereby allowing the sensor control device carrier 13 to move from the distal end to the proximal end.

In some embodiments, the second locking mechanism 111 and the third triggering mechanism 112 may be disposed on the inner wall of the housing 11. The second locking mechanism 111 may be disposed corresponding to the first locking mechanism 131, and the third triggering mechanism 112 may be disposed corresponding to the second triggered mechanism 123.

Referring to FIG. 10B, in some embodiments, the housing 11, the trigger member 12, and the sensor control device carrier 13 may be arranged in sequence in a nested configuration, with the housing 11 positioned outermost for being applied force. In some embodiments, the first triggering mechanism 121 and the first triggered mechanism 132 may be correspondingly disposed and located directly below the second locking mechanism 111 and the first locking mechanism 131. In this configuration, when the first triggered mechanism 132 is triggered by the first triggering mechanism 121 and moves inward, it causes the first locking mechanism 131 to move inward, thereby releasing the first locking mechanism 131 from its locked state with the second locking mechanism 111. This enhances triggering effectiveness and simplifies the internal space of the sensor applicator 10.

In some embodiments, there may be a plurality of second locking mechanisms 111. For example, there may be two second locking mechanisms 111 distributed on the front and rear surfaces of the inner wall of the housing 11.

In some embodiments, there may be a plurality of third triggering mechanisms 112. For example, there may be two third triggering mechanisms 112 distributed on the left and right surfaces of the inner wall of the housing 11.

In some embodiments, the second locking mechanism 111 may be a protrusion formed on the inner wall of the housing 11. The size and shape of the protrusion should allow it to securely engage with the first locking mechanism 131.

In some embodiments, referring to FIGS. 8A and 11, the third triggering mechanism 112 may include a second locking structure 1121 and a second sliding structure 1122. The second locking structure 1121 may be configured to lock with the first locking structure 1231, thereby locking the housing 11 and the trigger member 12; the second sliding structure 1122 may be configured to unlock the housing 11 and the trigger member 12 by sliding relative to the first sliding structure 1232.

In some embodiments, the second locking structure 1121 and the second sliding structure 1122 may be two adjacent protrusions arranged vertically, with the second locking structure 1121 being a lower protrusion and the second sliding structure 1122 being an upper protrusion.

In some embodiments, the first locking structure 1231 may engage with the lower protrusion to lock with it.

In some embodiments, a surface of the first sliding structure 1232 corresponding to the upper protrusion may be an inclined surface or a smooth surface. This enables relative movement between the first sliding structure 1232 and the upper protrusion.

Referring to FIGS. 8A, 8B, and 11, in some embodiments, a fifth guide rail 1242 (refer to FIGS. 8A and 8B) may be provided on the outer wall of the third main body part 124, and a sixth guide rail 113 (refer to FIG. 11) corresponding to the fifth guide rail 1242 may be provided on the inner wall of the housing 11. This configuration enables stable relative movement between the housing 11 and the trigger member 12, enhancing the stability and effectiveness of triggering by the trigger member 12.

In some embodiments, the number of fifth guide rails 1242 may be equal to the number of sixth guide rails 113. In some embodiments, the fifth guide rails 1242 may be uniformly distributed on the outer wall of the third main body part 124. In some embodiments, the sixth guide rails 113 may be uniformly distributed on the inner wall of the housing 11. This further enhances the stability of movement between the housing 11 and the trigger member 12.

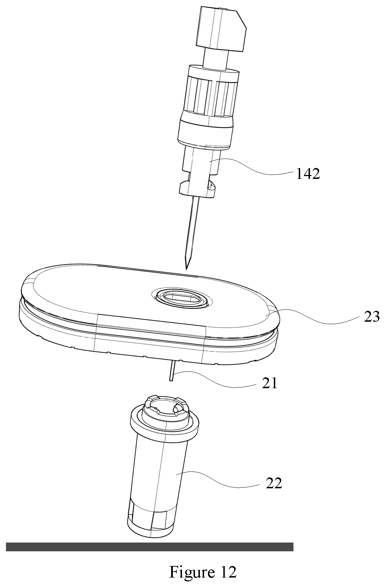

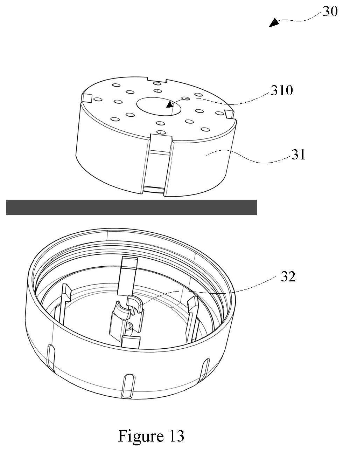

FIG. 12 is an exploded schematic view of a sealing assembly according to an example of the present disclosure. FIG. 13 is an exploded schematic view of the sensor applicator cap 30 according to an example of the present disclosure.

The components shown in FIG. 12 collectively form the sealing assembly. Referring to FIG. 12, in some embodiments, the sealing assembly may include the sensor control device 20, the needle-shaped part 1421, and a sensor cap 22. The sensor control device 20 may include an outer shell 23 and a sensor 21. The needle-shaped part 1421, the outer shell 23, and the sensor cap 22 may be connected to form a sealed space that encloses the sensor 21. In this configuration, since the sensor 21 needs to be implanted into a host, it may need to undergo sterilization prior to implantation, and it may need to be ensured that the sensor 21 remains in a sterile environment before implantation. The sealed space formed by the needle-shaped part 1421, the outer shell 23, and the sensor cap 22 ensures that the sensor 21 is maintained in a sterile environment before implantation, thereby guaranteeing the harmlessness of the implantation procedure.

In some embodiments, an adhesive patch may be provided at the bottom of the outer shell 23. The adhesiveness of the patch enables more secure attachment of the sensor control device 20 to the host's body surface.

In some embodiments, the sensor control device 20 may include functional components such as a signal processor, a signal transmitter, and a power source disposed within the outer shell 23. In this configuration, the signal processor can analyze and process physiological information of the host detected by the sensor 21, and the signal transmitter can send the physiological signals processed by the signal processor about the host to the reading device 2.

Referring to FIG. 13, in some embodiments, the sensor applicator cap 30 may include a decapping structure 32 and a drying member 31. The decapping structure 32 is configured such that when an operator removes the sensor applicator cap 30, the sensor cap 22 is removed simultaneously via the decapping structure 32. This improves the operator experience.

In some embodiments, the drying member 31 may be configured to maintain a dry environment within the internal space formed by the housing 11 and the sensor applicator cap 30. This reduces the impact of humidity on the performance of electronic components within the outer shell 23 and the sensor 21, etc, thereby extending their shelf life.

In some embodiments, the drying member 31 may have a fourth through hole 310. The fourth through hole 310 allows the sensor cap 22 to pass through and engage with the decapping structure 32.

The present disclosure provides a sensor applicator 10 capable of improving triggering effectiveness and the operator experience.

Although the present disclosure has been described in detail with reference to the accompanying drawings and examples, it should be understood that these descriptions are not intended to limit the present disclosure in any form. Those skilled in the art can make modifications and variations to the present disclosure as needed without departing from the spirit and scope of the present disclosure, and these modifications and variations all fall within the scope of the present disclosure.

## Claims

1. A sensor applicator, **characterized in that** the sensor applicator is configured to apply a sensor control device to a host, the sensor applicator having a proximal end and a distal end and comprising a housing, a sensor control device carrier, a sharp module, and a trigger member; wherein the sensor control device carrier is configured to releasably receive the sensor control device; the sharp module is configured to releasably apply the sensor control device to the host; the trigger member is configured to, when triggered by the housing, cause the sensor control device carrier to drive the sharp module and the sensor control device to move from the distal end to the proximal end; and the sharp module is further configured to, when moving a predetermined distance relative to the trigger member under the drive of the sensor control device carrier, be triggered by the trigger member and move from the proximal end to the distal end.

2. The sensor applicator according to claim 1, **characterized in that** it comprises a first driving member configured to drive the sensor control device carrier to move from the distal end to the proximal end when the trigger member is triggered by the housing.

3. The sensor applicator according to claim 1, **characterized in that** it comprises a second driving member configured to drive the sharp module to move from the proximal end to the distal end when the sharp module has moved the predetermined distance relative to the trigger member and is triggered by the trigger member.

4. The sensor applicator according to claim 1 or 2, **characterized in that** the sensor control device carrier comprises a first locking mechanism and a first triggered mechanism, wherein the first locking mechanism is configured to be locked with the housing in an initial state, and the first triggered mechanism is configured to, when triggered by the trigger member, cause the sensor control device carrier to move from the distal end to the proximal end.

5. The sensor applicator according to claim 1 or 3, **characterized in that** the sensor control device carrier comprises a first limiting mechanism, and the sharp module comprises a first retaining mechanism, wherein the first limiting mechanism is configured to restrict movement of the sharp module relative to the sensor control device carrier by engaging with the first retaining mechanism.

6. The sensor applicator according to claim 4, **characterized in that** the trigger member comprises a first triggering mechanism configured to trigger the first triggered mechanism when the trigger member is triggered by the housing.

7. The sensor applicator according to claim 5, **characterized in that** the trigger member comprises a second triggering mechanism configured to trigger the first retaining mechanism when the sharp module has moved the predetermined distance.

8. The sensor applicator according to claim 6, **characterized in that** the trigger member comprises a second triggered mechanism configured to be locked with the housing in the initial state and, when triggered by the housing, cause the first triggering mechanism to trigger the first triggered mechanism.

9. The sensor applicator according to claim 1, 7, or 8, **characterized in that** the trigger member extends beyond the housing and is in a first position in the initial state, the housing is in a second position in the initial state, and the housing triggers the trigger member by moving from the second position to the first position.

10. The sensor applicator according to claim 8, **characterized in that** the housing comprises a second locking mechanism and a third triggering mechanism, wherein the second locking mechanism is configured to be locked with the first locking mechanism in the initial state, and the third triggering mechanism is configured to be locked with the second triggered mechanism in the initial state and trigger the second triggered mechanism when the housing moves relative to the trigger member.
